# EUROPEAN PATENT APPLICATION

(11) **EP 3 998 352 A1**
(43) Date of publication of application: **18.05.2022**
(21) Application number: 19926362.5
(22) Date of filing: 01.11.2019
(51) Int. Cl.: C12Q 1/6858, C12Q 1/6844, G01N 21/64

(54) **DETECTION TECHNOLOGY SYSTEM FOR ENRICHING LOW-ABUNDANCE DNA MUTATION ON THE BASIS OF NUCLEASE-COUPLED PCR PRINCIPLE AND APPLICATION THEREOF**

(30) Priority: 22.04.2019 CN 201910324580
(71) Applicant: Jiaohong Biotechnology (Shanghai) Co., Ltd., Minhang District Shanghai 201109 (CN)
(72) Inventor: FENG, Yan, Shanghai 201109 (CN); LIU, Qian, Shanghai 201109 (CN); XUN, Guanhua, Shanghai 201109 (CN); GUO, Xiang, Shanghai 201109 (CN); LI, Zhonglei, Shanghai 201109 (CN); CHONG, Yuesheng, Shanghai 201109 (CN)
(74) Representative: Mewburn Ellis LLP
(86) International application number: PCT/CN2019/115151
(87) International publication number: WO 2020/215652

(57) **Abstract**

A detection system for enriching a low-abundance single-nucleotide mutant gene on the basis of nuclease-coupled PCR principle. Also provided is a method for increasing relative abundance of a target nucleic acid, comprising: (a) providing a nucleic acid sample containing the target nucleic acid and a non-target nucleic acid, the target nucleic acid having abundance of F1a in the nucleic acid sample; and (b) performing PCR and a nucleic acid cleavage reaction in an amplification-cleavage reaction system using the nucleic acids in the nucleic acid sample as templates to obtain an amplification-cleavage reaction product, the target nucleic acid having abundance of F1b in the amplification-cleavage reaction product, and the ratio of F1b/F1a being ≥ 10.

Sequence listing

<110>  JIAOHONG BIOTECHNOLOGY (SHANGHAI) CO., LTD.

<120>  DETECTION TECHNOLOGY SYSTEM FOR ENRICHING LOW-ABUNDANCE DNA MUTATION
ON THE BASIS OF NUCLEASE-COUPLED PCR PRINCIPLE AND APPLICATION THEREOF

<130>  P2019-1785

<150>  CN 201910324580.2
<151>  2019-04-22

<160>  40

<170>  PatentIn version 3.5

<210>  1
<211>  158
<212>  DNA
<213>  artificial sequence

<220>
<223>   KRAS G12D(wt)

<400>  1
gtgacatgtt ctaatatagt cacattttca ttatttttat tataaggcct gctgaaaatg      60

actgaatata aacttgtggt agttggagct ggtggcgtag gcaagagtgc cttgacgata     120

cagctaattc agaatcattt tgtggacgaa tatgatcc                            158


<210>  2
<211>  158
<212>  DNA
<213>  artificial sequence

<220>
<223>  KRAS G12D(mut)

<400>  2
gtgacatgtt ctaatatagt cacattttca ttatttttat tataaggcct gctgaaaatg      60

actgaatata aacttgtggt agttggagct gatggcgtag gcaagagtgc cttgacgata     120

cagctaattc agaatcattt tgtggacgaa tatgatcc                            158


<210>  3
<211>  21
<212>  DNA
<213>  artificial sequence

<220>
<223>  KRAS G12D-158F

<400>  3
gtgacatgtt ctaatatagt c                                               21


<210>  4
<211>  20
<212>  DNA

<213>  artificial sequence

<220>
<223>  KRAS G12D-158R

<400>  4
ggatcatatt cgtccacaaa                                                    20


<210>  5
<211>  16
<212>  DNA
<213>  artificial sequence

<220>
<223>  KRAS G12D-GF1

<400>  5
tttggagcta gtggcg                                                        16


<210>  6
<211>  16
<212>  DNA
<213>  artificial sequence

<220>
<223>  KRAS G12D-GR1

<400>  6
tctacgccac aagctc                                                        16


<210>  7
<211>  20
<212>  DNA
<213>  artificial sequence

<220>
<223>  KRAS G12D-7PF

<400>  7
aggcctgctg aaaatgactg                                                    20


<210>  8
<211>  21
<212>  DNA
<213>  artificial sequence

<220>
<223>  KRAS G12D-7PR

<400>  8
gctgtatcgt caaggcactc t                                                  21


<210>  9
<211>  17
<212>  DNA
<213>  artificial sequence

<220>

<223>  KRAS G12D(wt)-7P

<400>  9
ttggagctgg tggcgta                                                    17


<210>  10
<211>  17
<212>  DNA
<213>  artificial sequence

<220>
<223>  KRAS G12D(mut)-7P

<400>  10
ttggagctga tggcgta                                                    17


<210>  11
<211>  139
<212>  DNA
<213>  artificial sequence

<220>
<223>  PIK3CA E545K(wt)

<400>  11
gagacaatga attaagggaa aatgacaaag aacagctcaa agcaatttct acacgagatc      60

ctctctctga aatcactgag caggagaaag attttctatg gagtcacagg taagtgctaa     120

aatggagatt ctctgtttc                                                  139


<210>  12
<211>  139
<212>  DNA
<213>  artificial sequence

<220>
<223>  PIK3CA E545K(mut)

<400>  12
gagacaatga attaagggaa aatgacaaag aacagctcaa agcaatttct acacgagatc      60

ctctctctga aatcactaag caggagaaag attttctatg gagtcacagg taagtgctaa     120

aatggagatt ctctgtttc                                                  139


<210>  13
<211>  20
<212>  DNA
<213>  artificial sequence

<220>
<223>  PIK3CA E545K-139F

<400>  13
gagacaatga attaagggaa                                                 20


<210>  14

<211>  19
<212>  DNA
<213>  artificial sequence

<220>
<223>  PIK3CA E545K-139R

<400>  14
gaaacagaga atctccatt                                                    19


<210>  15
<211>  16
<212>  DNA
<213>  artificial sequence

<220>
<223>  PIK3CA E545K-GF1

<400>  15
tgaaatcacc gagcag                                                       16


<210>  16
<211>  16
<212>  DNA
<213>  artificial sequence

<220>
<223>  PIK3CA E545K-GR1

<400>  16
ttctcctgcg cagtga                                                       16


<210>  17
<211>  25
<212>  DNA
<213>  artificial sequence

<220>
<223>  PIK3CA E545K-4PF

<400>  17
gaacagctca aagcaatttc tacac                                             25


<210>  18
<211>  23
<212>  DNA
<213>  artificial sequence

<220>
<223>  PIK3CA E545K-4PR

<400>  18
agcacttacc tgtgactcca tag                                               23


<210>  19
<211>  19
<212>  DNA
<213>  artificial sequence

<220>
<223>  PIK3CA E545K(wt)-4P

<400>  19
ctgaaatcac tgagcagga                                                    19


<210>  20
<211>  20
<212>  DNA
<213>  artificial sequence

<220>
<223>  PIK3CA E545K(mut)-4P

<400>  20
tctgaaatca ctaagcagga                                                   20


<210>  21
<211>  157
<212>  DNA
<213>  artificial sequence

<220>
<223>  EGFR delE746-A750(wt)

<400>  21
ctgtcatagg gactctggat cccagaaggt gagaaagtta aaattcccgt cgctatcaag      60

gaattaagag aagcaacatc tccgaaagcc aacaaggaaa tcctcgatgt gagtttctgc     120

tttgctgtgt gggggtccat ggctctgaac ctcaggc                              157


<210>  22
<211>  142
<212>  DNA
<213>  artificial sequence

<220>
<223>  EGFR delE746-A750 (mut)

<400>  22
ctgtcatagg gactctggat cccagaaggt gagaaagtta aaattcccgt cgctatcaaa      60

acatctccga aagccaacaa ggaaatcctc gatgtgagtt tctgctttgc tgtgtggggg     120

tccatggctc tgaacctcag gc                                              142


<210>  23
<211>  20
<212>  DNA
<213>  artificial sequence

<220>
<223>  EGFR  delE746-157F

<400>  23
ctgtcatagg gactctggat                                                   20

<210>  24
<211>  19
<212>  DNA
<213>  artificial sequence

<220>
<223>  EGFR delE746-157R

<400>  24
gcctgaggtt cagagccat                                                    19


<210>  25
<211>  16
<212>  DNA
<213>  artificial sequence

<220>
<223>  EGFR  delE746-A750-GF1

<400>  25
taaggaatta agagaa                                                       16


<210>  26
<211>  15
<212>  DNA
<213>  artificial sequence

<220>
<223>  EGFR delE746-A750-GR1

<400>  26
ttgcttctct taatt                                                        15


<210>  27
<211>  19
<212>  DNA
<213>  artificial sequence

<220>
<223>  EGFR  delE746-A750-4PF

<400>  27
ccagaaggtg agaaagtta                                                    19


<210>  28
<211>  18
<212>  DNA
<213>  artificial sequence

<220>
<223>  EGFR  delE746-A750-4PR

<400>  28
tcgaggattt ccttgttg                                                     18


<210>  29
<211>  27

<212>  DNA
<213>  artificial sequence

<220>
<223>  EGFR  delE746-A750(wt)-4P

<400>  29
cttctcttaa ttccttgata gcgacgg                                          27


<210>  30
<211>  25
<212>  DNA
<213>  artificial sequence

<220>
<223>  EGFR  delE746-A750(mut)-4P

<400>  30
cgctatcaaa acatctccga aagcc                                            25


<210>  31
<211>  154
<212>  DNA
<213>  artificial sequence

<220>
<223>  NRAS A59T  (wt)

<400>  31
ccaggattct tacagaaaac aagtggttat agatggtgaa acctgtttgt tggacatact      60

ggatacagct ggacaagaag agtacagtgc catgagagac caatacatga ggacaggcga     120

aggcttcctc tgtgtatttg ccatcaataa tagc                                 154


<210>  32
<211>  154
<212>  DNA
<213>  artificial sequence

<220>
<223> NRAS A59T  (mut)

<400>  32
ccaggattct tacagaaaac aagtggttat agatggtgaa acctgtttgt tggacatact      60

ggatacagct ggaaaagaag agtacagtgc catgagagac caatacatga ggacaggcga     120

aggcttcctc tgtgtatttg ccatcaataa tagc                                 154


<210>  33
<211>  24
<212>  DNA
<213>  artificial sequence

<220>
<223>  NRAS A59T-154F

<400>  33

ccaggattct tacagaaaac aagt                                              24


<210>  34
<211>  25
<212>  DNA
<213>  artificial sequence

<220>
<223>  NRAS A59T-154R

<400>  34
gctattattg atggcaaata cacag                                             25


<210>  35
<211>  16
<212>  DNA
<213>  artificial sequence

<220>
<223>  NRAS A59T-GF1

<400>  35
ttggatacag ttggac                                                       16


<210>  36
<211>  16
<212>  DNA
<213>  artificial sequence

<220>
<223>  NRAS A59T-GR1

<400>  36
tcttgtccat ctgtat                                                       16


<210>  37
<211>  23
<212>  DNA
<213>  artificial sequence

<220>
<223>  NRAS A59T-PF

<400>  37
gatggtgaaa cctgtttgtt gga                                               23


<210>  38
<211>  21
<212>  DNA
<213>  artificial sequence

<220>
<223>  NRAS A59T-PR

<400>  38
tcgcctgtcc tcatgtattg g                                                 21

<210>  39
<211>  18
<212>  DNA
<213>  artificial sequence

<220>
<223>  NRAS A59T(wt)-P

<400>  39
ctggatacag ctggacaa                                                    18


<210>  40
<211>  20
<212>  DNA
<213>  artificial sequence

<220>
<223>  NRAS A59T(mut)-P

<400>  40
tactggatac aactggacaa                                                  20

## Description

### Technical field

The present invention belongs to the field of biotechnology, and specifically relates to a detection system based on the principle of nuclease-coupled PCR to enrich low-abundance single nucleotide variant genes (single nucleotide variant, SNV).

### Background

In recent years, the concept of "liquid biopsy" is emerging. The basic idea is to use blood and other body fluid samples to replace tumor tissue samples for pathological and molecular biological testing, and it has become a trend to obtain tumor gene mutation information by detecting circulating tumor DNA in patients' body fluid samples (mainly blood). Compared with the current standard tissue biopsy, the revolutionary liquid biopsy has the following irreplaceable advantages: small trauma, repeatability, homogenization and heterogeneity, real-time judgment of curative effect, and dynamic adjustment of treatment decisions with the development of the tumor. Therefore, the top ten breakthrough technologies of the year (Breakthrough Technologies 2015) released by MIT Technology Review in 2015, the expectations for the next ten years in ASCO's annual progress (Clinical cancer advance 2015), liquid biopsy are all on the list. By detecting ctDNA to track the specific genetic changes of the tumor throughout the course of the disease, it is of great value for tumor screening, diagnosis, efficacy monitoring, and prognostic judgment. At the same time, it can explore the molecular mechanism of tumor metastasis, recurrence and drug resistance, and identify new targeted treatment sites, etc.. Therefore, ctDNA detection has become one of the three popular directions for tumor liquid biopsy applications.

There are small fragments of free DNA (cell-free DNA, cfDNA) in the blood, which come from dead cells. Usually dead cells will be eliminated, so that the content of cfDNA is very low, usually a healthy person contains 25ng cfDNA in 1mL of plasma. The content of cfDNA in cancer patients is several times higher than normal, part of which is ctDNA (circulating tumor DNA). The relative content of ctDNA is related to tumor load and response to treatment, and can be used to identify driver genes, guide clinical treatment, monitor clinical treatment effects and cancer recurrence, reveal treatment resistance, and detect disease progression. In some respects, the sensitivity of the ctDNA method is even higher than that of traditional methods. For example, compared with traditional imaging tests, tracking the tumor DNA in the blood of patients with early breast cancer after surgery can detect breast cancer recurrence 7.9 months earlier. The detection of KRAS mutations of cfDNA in lung cancer and intestinal cancer also has important diagnostic value for lung cancer. ctDNA can be detected in the early stages of cancer. Because cfDNA is easy to be collected and has been shown to be highly consistent with the variation in tissues in lung cancer, liquid biopsy of ctDNA has attracted more and more attention.

Although circulating tumor DNA is a good alternative sample of tumor tissue, the detection of circulating tumor DNA requires extremely sensitive technology due to the scarce content of circulating tumor DNA. The application of BEAMing amplification method greatly improves the sensitivity of DNA detection technology. In 2007, the developers of this technology, Bert Vogelstein and Kenneth Kinzler of Johns Hopkins University in the United States, tracked the circulating tumor DNA of 18 patients with colorectal cancer. Studies have shown that patients whose circulating tumor DNA can still be detected after surgery have basically relapsed. In patients whose circulating tumor DNA is not detected after surgery, there is no recurrence of colorectal cancer, which shows the good clinical application prospects of circulating tumor DNA. The sensitivity of BEAMing technology is high, which can reach 0.1% to 0.01%. It is an ideal technique for detecting circulating tumor DNA. However, due to its complicated operation and expensive equipment, it is not suitable for large-scale clinical promotion.

The current rare mutation detection methods mainly include gene sequencing as the "gold standard". However, the sensitivity of sequencing is limited. In the context of a large number of wild-type genes, sequencing can only detect 20% of mutations, which will lead to false negative results and take a long time. Compared with sequencing, the sensitivity of denaturing high performance liquid chromatography has been improved, but it requires PCR post-processing, which can easily cause laboratory contamination, easily lead to false positive results, specificity is also limited, and the operation steps are complicated and the cycle is long. Detection methods based on the principle of nucleic acid hybridization, such as TaqMan probes, have a selective detection level equivalent to sequencing methods. Amplification refractory mutation system (ARMS) is a commonly used method for detecting rare mutations. Based on the distinguishing ability of different mismatched bases at the 3' end of the primer, the mutant template is specifically selected and amplified, but due to limited distinguishing ability, the selectivity is general and different types of mutations are quite different. In 2011, Life Technology has developed a highly selective mutation detection technology-cast PCR technology, which is based on ARMS technology and uses the high specificity of MGB probes to further improve the selectivity of the reaction. However, the synthesis of MGB probes is difficult and expensive, which is not conducive to wide application. Digital PCR is another high-sensitivity detection technology that has emerged in recent years. This technology can reach a sensitivity of 0.01% at the highest, but this technology is prone to false positive results. Similarly, high equipment and reagent prices, and extremely high experimental operation requirements also limit its large-scale promotion.

Therefore, there is an urgent need in the art to develop a method for enriching and detecting mutant DNA with high specificity, high sensitivity and low abundance.

### Summary of the invention

The purpose of the present invention is to provide a method for enriching and detecting mutant DNA with high specificity, high sensitivity and low abundance.

In a first aspect of the present invention, it provides a method for increasing the relative abundance of target nucleic acid, comprising the steps:
(a) providing a nucleic acid sample, the nucleic acid sample contains a first nucleic acid and a second nucleic acid, wherein the first nucleic acid is a target nucleic acid and the second nucleic acid is a non-target nucleic acid,
   and, the abundance of the target nucleic acid in the nucleic acid sample is F1a;
(b) performing a polymerase chain reaction (PCR) and nucleic acid cleavage reaction in an amplification-cleavage reaction system using the nucleic acid in the nucleic acid sample as a template, thereby obtaining an amplification-cleavage reaction product;
wherein, the nucleic acid cleavage reaction is used to specifically cleave the non-target nucleic acid, but not the target nucleic acid;
in addition, the amplification-cleavage reaction system contains (i) a reagent required for PCR reaction and (ii) a reagent required for nucleic acid cleavage reaction;
wherein, the abundance of the target nucleic acid in the amplification-cleavage reaction product is Fib,
wherein the ratio of F1b/ F1a is ≥ 10.

In another preferred embodiment, the target nucleic acid and the non-target nucleic acid differ by only one base.

In another preferred embodiment, when 1%≤F1a≤10%, the ratio of F1b/F1a is ≥ 10, when 0.1%≤F1a≤0.5%, the ratio of F1b/F1a is ≥ 100, when F1a≤0.1%, the ratio of F1b/F1a is ≥200.

In another preferred embodiment, the nucleic acid sample includes a nucleic acid sample that is directly heated and lysed, a nucleic acid sample that is directly treated with a lyase protease, a nucleic acid sample that has been extracted, a nucleic acid sample that has been pre-amplified by PCR, or any sample containing nucleic acid.

In another preferred embodiment, the nucleic acid sample that has been pre-amplified by PCR is a PCR amplified product of 1-30 cycles, preferably 10-20 cycles, and more preferably 15-30 cycles.

In another preferred embodiment, the target nucleic acid is a nucleotide sequence containing a mutation.

In another preferred embodiment, the mutation is selected from the group consisting of nucleotide insertion, deletion, substitution, and a combination thereof.

In another preferred embodiment, the mutation includes SNV.

In another preferred embodiment, the non-target nucleic acid (or a second nucleic acid) is a wild-type nucleotide sequence, a highly abundant nucleotide sequence, and a combination thereof.

In another preferred embodiment, the abundance of the non-target nucleic acid in the nucleic acid sample is F2a.

In another preferred embodiment, F1a+F2a=100%.

In another preferred embodiment, the ratio of F2a/F1a is ≥20, preferably ≥ 50, more preferably ≥ 100, and most preferably ≥ 1000 or ≥ 5000.

In another preferred embodiment, the abundance of the non-target nucleic acid in the amplification-cleavage reaction product is F2b.

In another preferred embodiment, F1b+F2b=100%.

In another preferred embodiment, F1b/F2b ≥ 0.5, preferably ≥ 1, more preferably ≥ 2, and most preferably ≥ 3 or ≥ 5.

In another preferred embodiment, the ratio of F1b/F1a is ≥ 200, preferably ≥ 500, more preferably ≥ 1000, and most preferably ≥ 2000 or ≥ 5000 or higher.

In another preferred embodiment, F1a ≥ 0.5%, preferably ≤ 0.2%, more preferably ≤ 0.1%, and most preferably ≤ 0.01%.

In another preferred embodiment, F1b ≥ 10%, preferably ≥ 30%, more preferably ≥ 50%, and most preferably ≤ 70%.

In another preferred embodiment, "a reagent required for PCR reaction" includes: DNA polymerase.

In another preferred embodiment, "a reagent required for PCR reaction" further includes: dNTP, 1-5 Mm Mg²⁺, PCR buffer.

In another preferred embodiment, "a reagent required for nucleic acid cleavage reaction" includes: a nucleic acid cleavage tool enzyme and guide DNA (gDNA).

In another preferred embodiment, the nucleic acid cleavage tool enzyme is a high-temperature stable double-stranded DNA cleavage tool enzyme.

In another preferred embodiment, the nucleic acid cleavage tool enzyme is selected from but not limited to the following Argonaute proteins and the mutants thereof from Thermophiles (≥ 60° C): *Pf*Ago (*Pyrococcus furiosus* Ago), *Mƒ*Ago (*Methanocaldococcus fervens* Ago), *Tc*Ago (*Thermogladius calderae* Ago), *Tf*Ago (*Thermus ƒiliƒormis* Ago), *Aa*Ago (*Aquifex aeolicus* Ago), etc..

In another preferred embodiment, the nucleic acid cleavage tool enzyme is *PfAgo.*

In another preferred embodiment, the gDNA forms a complex with the nucleic acid cleavage tool enzyme, and the complex specifically cleaves non-target nucleic acids.

In another preferred embodiment, the gDNA and the nucleic acid sequence in the target region of the target nucleic acid (i.e., a first nucleic acid) form a first complementary binding region; and the gDNA also forms a second complementary binding region with the nucleic acid sequence of the target region of the non-target nucleic acid (i.e., a second nucleic acid).

In another preferred embodiment, the first complementary binding region contains at least 2 mismatched base pairs.

In another preferred embodiment, the second complementary binding region contains 0 or 1 mismatched base pair.

In another preferred embodiment, the second complementary binding region contains 1 mismatched base pair.

In another preferred embodiment, the first complementary binding region contains at least 2 mismatched base pairs, thereby causing the complex not to cleave the target nucleic acid; and the second complementary binding region contains 1 mismatched base pair, thereby causing the complex to cleave the non-target nucleic acid.

In another preferred embodiment, the targeted region of the target nucleic acid (i.e., a first nucleic acid) corresponds to the targeted region of the non-target nucleic acid (i.e., a second nucleic acid).

In another preferred embodiment, the length of the gDNA is 15-30 nt.

In another preferred embodiment, the position 7 and/or 10 of the gDNA are mismatched bases, and the mismatched bases are used to form mismatched base pairs in both the first complementary binding region and the second complementary binding region.

In another preferred embodiment, positions 2-8 of the gDNA are "seed region" regions, and positions 10 and 11 are cleavage key sites of *Pf*Ago.

In another preferred embodiment, the ratio (molar ratio) of the nucleic acid cleavage tool enzyme and gDNAs is 1:2 to 1:20.

In another preferred embodiment, in the amplification-cleavage reaction system, the nucleic acid cleavage tool enzyme is 30 nM, and the DNA polymerase is a high temperature resistant polymerase, preferably, Taq DNA polymerase, LA Taq DNA polymerase , Tth DNA polymerase, Pfu DNA polymerase, Phusion DNA polymerase, KOD DNA polymerase, etc., more preferably, 2X PCR Precision^{TM} Master Mix.

In another preferred embodiment, in the amplification-cleavage reaction system, the amount of nucleic acid used as a template is 0.1-100 nM.

In another preferred embodiment, the method further includes:
(c) detecting the amplification-cleavage reaction product, thereby determining the presence and/or quantity of the target nucleic acid.

In another preferred embodiment, the detection in step (c) includes quantitative detection, qualitative detection, or a combination thereof.

In another preferred embodiment, the quantitative detection is selected from the group consisting of: q-PCR, ddPCR, chemiluminescence, high resolution melting curve method, Sanger sequencing, NGS and the like.

In another preferred embodiment, the first nucleic acid includes n different nucleic acid sequences, wherein n is a positive integer ≥ 1.

In another preferred embodiment, n is 1, 2, 3, 4, 5, 6, 7, 8, 9, 10, 11, 12, 13, 14, 15, 16, 17, 18, 19, 20, 21 , 22, 23, 24, 25, 26, 27, 28, 29, 30, 31, 32, 33, 34, 35, 36, 37, 38, 39, 40, 41, 42, 43, 44, 45, 46 , 47, 48, 49, 50, 51, 52, 53, 54, 55, 56, 57, 58, 59, 60, 61, 62, 63, 64, 65, 66, 67, 68, 69, 70, 71 , 72, 73, 74, 75, 76, 77, 78, 79, 80, 81, 82, 83, 84, 85, 86, 87, 88, 89, 90, 91, 92, 93, 94, 95, 96 , 97, 98, 99, 100 or greater.

In another preferred embodiment, n is 2-1000, preferably 3-100, more preferably 3-50.

In another preferred embodiment, in step (b), C cycles of "high temperature denaturation-extension" are performed, wherein C is ≥ 5.

In another preferred embodiment, the high temperature denaturation temperature corresponds to the melting temperature of the DNA double strand of the PCR reaction and the cleavage temperature of the nucleic acid cleavage tool enzyme.

In another preferred embodiment, the high temperature denaturation temperature is 85-95°C.

In another preferred embodiment, the C is 5-35.

In another preferred embodiment, the method is non-diagnostic and non-therapeutic.

In another preferred embodiment, the nucleic acid sample includes a nucleic acid from a sample, wherein the sample is selected from the group consisting of blood, cells, serum, saliva, body fluid, plasma, urine, prostatic fluid, bronchial perfusate, cerebrospinal fluid, gastric juice, bile, lymphatic fluid, peritoneal fluid, feces, etc. and a combination thereof.

In a second aspect of the present invention, it provides an amplification-cleavage reaction system, which is used to simultaneously perform a polymerase chain reaction (PCR) and nucleic acid cleavage reaction on a nucleic acid sample, thereby obtaining an amplification-cleavage reaction product;
wherein, the nucleic acid sample contains a first nucleic acid and a second nucleic acid, wherein the first nucleic acid is a target nucleic acid, and the second nucleic acid is a non-target nucleic acid;
the nucleic acid cleavage reaction is used to specifically cut the non-target nucleic acid, but not the target nucleic acid;
the amplification-cleavage reaction system contains (i) a reagent required for PCR reaction and (ii) a reagent required for nucleic acid cleavage reaction.

In another preferred embodiment, the amplification-cleavage reaction system does not contain or contains the nucleic acid sample.

In another preferred embodiment, the concentration of Mn ions in the amplification-cleavage reaction system is 0.1-1 mM.

In another preferred embodiment, the concentration of Mg ions in the amplification-cleavage reaction system is 1-3 mM.

It should be understood that, within the scope of the present invention, each technical feature of the present invention described above and in the following (as examples) may be combined with each other to form a new or preferred technical solution, which is not listed here due to space limitations.

### Description of Figure

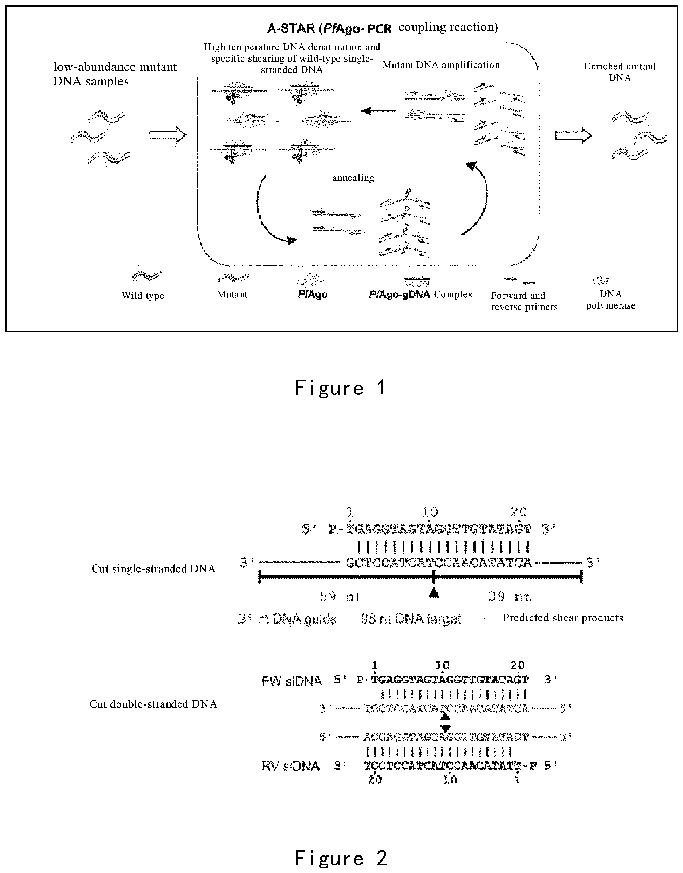

Figure 1 shows the schematic diagram of the technical solution of the present invention.
Figure 2 shows the recognition of single-stranded DNA (ssDNA) and double-stranded DNA (dsDNA) substrates by gDNA and the shearing mechanism of the PfAgo-gDNA complex.

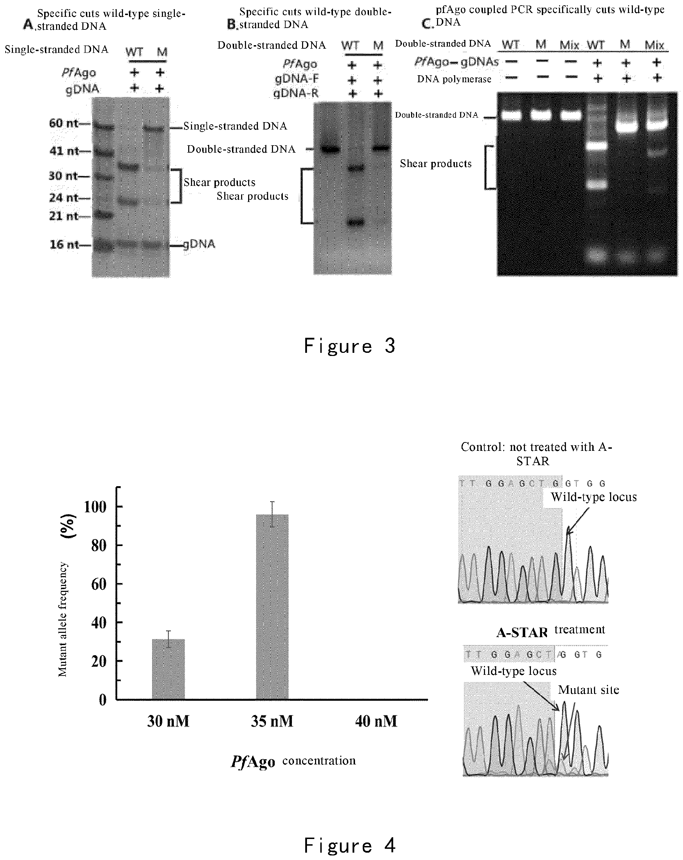

Figure 3 shows the differential shearing of ssDNA, dsDNA; and SNV of dsDNA under the PCR working system by the PfAgo-gDNA complex. Figure 3A-differential shearing of wild-type and mutant ssDNA substrates by PfAgo-gDNA complex under different forward and reverse gDNA combinations; Figure 3B-Distinguishing shearing of wild-type and mutant dsDNA substrates by PfAgo-gDNA complex under the preferred combination of forward and reverse gDNA; Figure 3C- under the preferred combination of forward and reverse gDNA, PfAgo-gDNA complex can distinguish shearing of wild-type and mutant dsDNA substrates and enriches mutant dsDNA under the PCR system.
Figure 4 shows the PfAgo-gDNA complex is optimized for the enrichment conditions of KRAS-G12D low-abundance mutant dsDNA substrates, and the optimal working concentration of PfAgo protein at a mutation ratio of 10 nM 1.0%.
Figure 5 shows the standard curve of the double TaqMan probe method for the detection of KRAS-G12D low-abundance mutant DNA substrates.
Figure 6 shows the high sensitivity detection of KRAS-G12D low-abundance mutant DNA (0.1%, 0.01%) substrates by the PfAgo-gDNA complex.
Figure 7 shows the high sensitivity detection and optimal enrichment results of the *Pƒ*Ago-gDNA complex on EGFR-delE746-A750 low-abundance mutant DNA (0.1%, 0.01%) substrates.
Figure 8 shows the high sensitivity detection and optimal enrichment results of *Pƒ*Ago-gDNA complex on KRAS-G12D, PIK3CA-E545K and EGFR-delE746-A750 triple low-abundance mutant DNA (0.01%) substrates.

### Detailed Description

After extensive and in-depth research, the present inventor has developed for the first time a method for enriching and detecting low-abundance mutant DNA with high sensitivity, good specificity, and high throughput. The overall technical system of the present invention is divided into three steps: PCR pre-amplification, Ago-PCR enrichment, and quantitative detection of target genes. Firstly, pre-treatment of samples from different sources, obtaining nucleic acid samples containing low-abundance target genes, and performing PCR pre-amplification to increase the molar concentration of target genes to meet the initial sample volume required for Ago-PCR enrichment. Secondly, performing specific enrichment and amplification of low-abundance mutant DNA, that is, the forward and reverse gDNAs, *Pf*Ago protein, PCR amplification system and pre-amplified PCR products are proportioned to prepare a low-abundance mutant DNA enrichment system, the enrichment system performs enrichment reaction while amplifying under specific conditions. *Pf*Ago specifically shears wild-type DNA under the guidance of gDNAs, thereby inhibiting its amplification, so as to achieve the purpose of enriching low-abundance mutant DNA. Finally, the target product enriched in the above system can be combined with multi-terminal detection equipment and methods, such as q-PCR, NGS, chemiluminescence method, high resolution melting curve method, Sanger sequencing, ddPCR, etc., to quantitatively detect the mutation of the target gene. The present invention has the advantages of non-invasiveness, easy operation, fast speed, etc., the sensitivity can reach 0.01%, the DNA amount of the sample can be as low as aM level, and it can better detect low-abundance mutant genes in human liquid biopsy. The technology of the present invention can be widely used in various fields of molecular diagnosis involving nucleic acid detection, such as tumor liquid biopsy, infectious diseases such as major infectious diseases and pathogenic infectious diseases (viruses, pathogenic bacteria) detection fields and other fields. The present invention has been completed on this basis.

### "A-STAR" detection technology

The core of the present invention is to develop a novel nucleic acid cleavage tool enzyme *Pf*Ago with single-point nucleic acid recognition specificity and high temperature stability, and to couple the PCR reaction to realize the cutting-while-amplification process, and establish "A-STAR (Ago-mediated Specific Target detection)" technology, the principle details are as follows: In the high temperature denaturation step of each cycle of PCR, dsDNA is denatured and melted into ssDNA. At this temperature, *Pf*Ago cleaves a pair of melting wild-type gene ssDNA under the guidance of a specially designed pair of gDNA. that is, this process can specifically cut the wild-type gene while retaining the mutant gene; in the subsequent PCR annealing step, the designed primers are located at least 20 nt upstream and downstream of the target nucleic acid SNV site, so that non-selective combination of wild-type genes and mutant genes; in the subsequent PCR extension step, since the wild-type gene has been cut at the mutation site, it cannot be used as a template for extension, while the mutant gene retains its original length and can be used as a template for amplification. Because this *Pf*Ago high-temperature specific cleavage and PCR amplification coupling reaction can be performed in each cycle of conventional PCR (20-35 cycles), it can realize cutting-while amplification to efficiently enrich low-abundance mutant genes. The technical advantages are: 1) differentiated shearing at high temperature, easy to operate; 2) gDNA sequence matches the target sequence, with high specificity; 3) it can be designed for any target sequence without sequence preference; 4) multiple detection of multiple nucleic acid targets by a single enzyme; 5) it can be combined with multi-terminal detection technology.

### "Coupling reaction of "PCR while cutting"

In the present invention, when the *Pƒ*Ago-gDNA complex is used for the "PCR while cutting" coupling reaction, the reaction can be carried out under appropriate conditions using the corresponding cleaving enzyme and the corresponding amplification enzyme, as long as the cleavage enzyme and amplification enzyme can perform their corresponding functions under this condition.

The research of the present invention shows that for the enrichment of mutant dsDNA signal through the coupling reaction, some key factors mainly include the following aspects:
① The initial template concentration in the enrichment reaction system: the total concentration of wild type (wt) and mutant type (mut) (nM~fM): preferably 0.1-100 nM.
② The initial *Pf*Ago protein concentration in the enrichment reaction system: preferably 20-100 nM;
③ Pre-processing time (minutes) of the *Pf*Ago-gDNA complex at 94°C: preferably 3-10 minutes;
(4) The initial gDNAs concentration in the enrichment reaction system: preferably 200-2000 nM;
⑤ The molar concentration ratio between *Pf*Ago protein and gDNAs: preferably 1:5~1:20;
⑥ Cycle number of enrichment PCR cycle: preferably 10-30;

In an embodiment of the present invention, KRAS-G12D wild and mutant fragments are used as substrates to experiment with the factors. The parameters can be seen in Table A.

**Table A**

| Table *Pf*Ago gDNAs-PCR coupling reaction key factors and parameters | | | | | | |
|---|---|---|---|---|---|---|
| Factors affecting the enrichment process | Parameters to be measured | | | | | |
| Template initial concentration (nM~fM) | 58nM | 10nM | 1.0nM | 10pM | 1.0pM | 100fM |
| Initial *Pf*Ago protein concentration (nM) | 20 | 40 | 60 | 80 | 100 | 120 |
| 94°C *Pf*Ago gDNAs pretreatment time (min) | 3 | 6 | 9 | 12 | 15 | 18 |
| Initial gDNAs concentration (nM) | 100 | 200 | 300 | 400 | 500 | 600 |
| The molar concentration ratio between *Pƒ*Ago protein and gDNAs | 1:2 | 1:3 | 1:4 | 1:6 | 1:8 | 1:10 |
| Cycle number of enrichment PCR | 10 | 15 | 20 | 25 | 30 | 40 |

### The main advantages of the present invention include:

1) The method of the present invention requires only a small amount of test samples, and has high detection sensitivity and accuracy;
2) The rapid detection technology of low-abundance mutant genes of the present invention can be used in the fields of early detection of trace nucleic acid markers of disease, dynamic monitoring of disease driver genes, and prognostic evaluation of certain diseases;
3) The method of the present invention can also be applied to the detection of infectious diseases, such as major infectious diseases and pathogenic infectious diseases, so as to achieve proactive management such as prediction and prevention.
4) The method of the present invention can also screen for genetic metabolic diseases related to mutant genes, and achieve proactive management such as prediction and early treatment.
5) The method of the present invention can also be used for screening of obstetrics and gynecology diseases, antenatal care and screening of genetic and metabolic diseases of newborns, so as to achieve effective measures such as prediction and early treatment.
6) The method of the present invention can also expand the detection of susceptibility genes, predict the risk of a small amount of disease in advance, and take effective preventive measures before the occurrence of the disease to minimize the possibility of disease suffering.

The present invention will be further explained below in conjunction with specific embodiments. It should be understood that these embodiments are only used to illustrate the present invention and not to limit the scope of the present invention. The experimental methods without specific conditions in the following examples usually follow the conventional conditions, such as the conditions described in Sambrook et al., Molecular Cloning: Laboratory Manual (New York: Cold Spring Harbor Laboratory Press, 1989), or according to manufacturing The conditions suggested by the manufacturer. Unless otherwise specified, percentages and parts are weight percentages and parts by weight.

### Example 1.

### Design of primers and detection probes

Primer design follows the principles: Primer requirements: ①The primer sequence should avoid a series of bases, especially a series of G; ②Tm is generally required to be 50~60°C; ③The ratio of (G+C)% is controlled at 28%~80%; ④ The last 5 bases at the 3' end of the primer cannot have more than 2 (G+C); ⑤ The closer the downstream primer is to the probe, the better, and the fragments can overlap. The amplified fragment is preferably 75-150 bp.

The principle that the detection probe design follows: the detection probe specifically binds to the target gene, and its binding site is in any region of the target gene. The 5' end of the probe is labeled with a fluorescent reporter (Reporter, R), such as FAM, VIC, etc., and the 3' end is labeled with a quencher (Quencher, Q). Probe design requirements: ①The 5' end of the probe cannot be G; ②The length of the probe should not be less than 13 bp; ③Avoiding a series of repeat base sequences; ④Tm is 65~70°C, the theoretical annealing temperature difference between the primer and the corresponding probe should preferably be 5~ 10°C; ⑤It is required that the SNV site to be detected is best located in the middle of the probe and as close to the 3' end as possible. If there is no suitable probe in the sequence of SNV that can achieve the required Tm value, the Quencher such as BHQ can be introduced into the 3' end.

In a specific embodiment, preferably, specific primer pairs designed for SNV mutation or fragment deletion mutation amplification designed for different circulating tumor DNA (ctDNA), the target genes include KRAS-G12D, PIK3CA-E545K, EGFR-delE746-A750, NRAS-A59T and other tumor mutant genes and their corresponding wild-type genes, which correspond to the primer pairs of group numbers in Table 7, respectively.

The present invention provides forward and reverse gDNAs and primers required for amplification of target genes designed for different target genes for SNV mutation or fragment deletion mutation enrichment, and specific detection probes, which include 4 groups of oligonucleotide sequences for enrichment and amplification of target genes similar to Table 1.

### Example 2.

### Design and optimization of oligonucleotide gDNAs

The core principle of the method for the enrichment of low-abundance mutant target genes is: on the one hand, the phosphorylation modification of the 5' end of gDNAs significantly improves the affinity of the *Pƒ*Ago-gDNA complex to nucleic acid substrates. At the same time, this method has found that there is a seed region on gDNA at the beginning of its establishment. The specificity of the interaction between the PfAgo-gDNA complex and the substrate is determined by the seed sequence in the gDNAs. This method explores different positions in the seed region of gDNAs (nucleotides on positions 2-15), and the effect of different nucleotides (bases) on improving the specific target binding of the PfAgo-gDNA complex to the target DNA substrate and its rules, analysis and summary of the design rules of gDNAs seed regions used to identify single nucleotide variations, as follows:
Principles followed in the design of gDNAs: forward and reverse oligonucleotide gDNAs sequence must be absolutely conservative. The gDNAs seed region spans the nucleotides on positions 2-15 of gDNA when targeting ss DNA. Its characteristics are similar to the seed regions reported by other Agos, and the nucleotides on positions 3, 6, 7, 9, 10 and 11 have the greatest impact on the specificity of PfAgo-gDNA complex binding to the target ssDNA substrate. Therefore, when designing gDNA, first optimizing the key nucleic acids and their positions that affect the substrate specificity in the gDNAs seed region to improve the specificity of the PfAgo-gDNA complex to single-stranded DNA substrates. According to the base difference between wild-type and mutant-type nucleotides of the target gene, by introducing several (more than 2) base permutations at positions 2-15 of gDNA, this programming method can distinguish ssDNA substrates with only a single nucleotide difference.

Secondly, in terms of the specific recognition of double-stranded DNA substrates, the appropriate forward and reverse gDNAs can be selected according to the specific distribution of PfAgo-gDNA complex to single-stranded DNA substrates, and testing the ability of the PfAgo-gDNA complex to distinguish substrates with only a single nucleotide difference under the double-gDNA mixed condition. Screening the highly specific PfAgo-gDNA complex for a specific nucleic acid substrate-for subsequent enrichment of low-abundance DNA mutations.

The specific oligonucleotide gDNA has its characteristics further including: 5' end and 3' end are modified with phosphate groups; gDNA length (≥ 15 nt), mismatch position, the influence of the mismatch sites and number introduced on gDNA on the specific recognition of nucleic acid substrates by the PfAgo-gDNA complex.

Preferably, when the 5' and 3' ends of the oligonucleotide gDNA are phosphorylated, and the additional mismatch sites introduced by the gDNA are located at the positions on 7, 10, and 11 of the gDNA, the enzyme has a good ability to distinguish substrate with only a single nucleotide difference, showing high-specific shearing of wild-type DNA, that is, it can distinguish wild-type and mutant target genes well.

In a specific embodiment, preferably, specific oligonucleotide gDNAs for SNV mutation or fragment deletion mutation enrichment designed for different circulating tumor DNA (ctDNA), including KRAS-G12D, PIK3CA-E545K, EGFR-delE746-A750, NRAS-A59T and other wild-type genes corresponding to multiple tumor mutant genes, which correspond to different gDNAs pairs in Table 6, respectively.

### Example 3.

### Differential shearing of ssDNA and dsDNA by PfAgo-gDNA complex

In this embodiment, it is mainly tested whether the PfAgo-gDNA complex still has a good discrimination and shearing ability for ssDNA, dsDNA; and dsDNA under the PCR working system under ordinary PCR reaction buffer and other components.

### 3.1 Method

The components and working conditions involved in this example mainly include: 2×PCR Taq Master Mix, forward and reverse primers, forward and reverse gDNAs, PfAgo, MnCl₂, templates (pure wild, pure mutation, and half wild and half mutant) and so on, as shown in Table 8.

Table 8 takes 25 µL system as an example, PfAgo enrichment reaction system components and preparation sequence.

The description of each component in Table 8 is as follows:
The 2× PCR Taq Master Mix reaction solution is made up of 2× PCR buffer, dNTPs and hot-start enzyme. 2× PCR buffer: KCl, (NH₄)₂SO₄, 3 mM MgCl₂, Tris-HCl, pH 8.3 (25°C). dNTPs include dATP, dGTP, dCTP and dTTP, and the final concentration in the reaction system is 0.4mM. The hot-start enzyme uses Taq DNA polymerase with a concentration of 5U/µL, and the final concentration in the reaction system is 0.1-0.5 U/µL. 2× PCR Taq Master Mix reaction solution is from abm biotechnology company (Item No: G013).

For the forward and reverse primers, the preferred primers corresponding to each target gene in Table 6 were used.

For the forward and reverse gDNAs, the preferred gDNAs pair corresponding to each target gene in Table 5 were used.

The concentration of the PfAgo mother solution stored after the pre-purification is 5µM. In actual use, it needs to be diluted with the prepared Reaction Buffer in advance under ice bath conditions: first diluting the 5µMPfAgo mother solution to 1µM, and then using Reaction Buffer to dilute it to 0.3µM. Reaction Buffer components: 15 mM Tris-Cl, 250 mM NaCl, pH 8.0.

The ssDNA and dsDNA in the template are 60 nt KRAS-G12D ssDNA wild-type and mutant fragments, and 620 bp KRAS-G12D dsDNA wild-type and mutant fragments, respectively.

The reaction conditions for the shearing of 60 nt KRAS-G12D ssDNA wild-type and mutant fragments by PfAgo-gDNA complex: After 15 minutes at 95°C, slowly lower the temperature to 10°C and keep it warm.

PfAgo-gDNA complex cleavage reaction of 620 bp KRAS-G12D dsDNA wild-type and mutant fragments and PCR working procedures:
The PCR reaction program of PfAgo enrichment system includes:

| step | Number of cycles | Temperature(°C) | Reaction time (Minutes: seconds) |
|---|---|---|---|
| 1 | 1 | 94 | 03:00 |
| 2 | 10-30 | 94 | 00:30 |
| | | 55 | 00:20 |
| | | 72 | 00:20 |
| 3 | 1 | 72 | 01:00 |

### 3.2 Results

As shown in Figure 3. The PfAgo-gDNA complex can distinguish the shearing of ssDNA, dsDNA; and SNV of dsDNA under the PCR working system.

### Example 4.

### Enrichment of mutant dsDNA by PfAgo-gDNA complex

In this embodiment, the PfAgo-gDNA complex is tested for the discrimination and shearing of wild-type and mutant dsDNA substrates and the enrichment of mutant dsDNA under the PCR system.

### 4.1 Method

According to the experimental steps of the low-abundance mutant DNA enrichment system described in the present invention, firstly, according to the sequence characteristics of the KRAS-G12D gene fragment, specific amplification primers, gDNAs and detection probes are designed and screened. See the sequence in Table 2 for details.

The gDNAs and primers were synthesized by Sangon Biotech (Shanghai) Co., Ltd.. KRAS gene gDNAs are phosphorylated at the 5' end.

Using KRAS-G12D wild and mutant fragments as substrates, analyzing the key factors that affect the signal enrichment of mutant dsDNA when the PfAgo-gDNA complex is used for PCR while shearing. See Table A for specific parameters.

This example optimizes the PfAgo-gDNA complex under the PCR system to distinguish shearing of wild-type and mutant dsDNA substrates and the enrichment conditions for mutant dsDNA. The components and working conditions involved in this example mainly include: 2×PCR Taq Master Mix, forward and reverse primers, forward and reverse gDNAs, PfAgo, MnCl₂, template (10nM 1.0% mut KRAS-G12D), etc., as shown in Table 8.

For the forward and reverse primers, the preferred primers corresponding to each target gene in Table 7 were used.

For the forward and reverse gDNAs, the preferred gDNAs pairs corresponding to each target gene in Table 6 were used.

The concentration of the PfAgo mother solution stored after the pre-purification is 5µM. In actual use, it needs to be diluted with the prepared Reaction Buffer in advance under ice bath conditions: first diluting the 5µMPfAgo mother solution to 1µM, and then using Reaction Buffer to dilute it to 0.3µM. Reaction Buffer 1 component: 15 mM Tris-Cl, 250 mM NaCl, pH 8.0.

Template: The prepared 10 nM 1.0% mut KRAS-G12D sample was quantified using the Pikogreen dsDNA quantification kit (super sensitive) (compatible with Qubit 3.0) sold by life ilab Bio.

PfAgo-gDNA complex cleavage reaction of 620 bp KRAS-G12D dsDNA wild-type and mutant fragments and PCR working procedures:
PfAgo's PCR reaction procedure for the enrichment of 10 nM 1.0% mut KRAS-G12D mutant gene includes:

| step | Number of cycles | Temperature (°C) | Reaction time (Minutes: seconds) |
|---|---|---|---|
| 1 | 1 | 94 | 03:00 |
| 2 | 10-30 | 94 | 00:30 |
| | | 55 | 00:20 |
| | | 72 | 00:20 |
| 3 | 1 | 72 | 01:00 |

After the PfAgo-gDNA complex distinguishes shearing of KRAS-G12D wild-type and mutant dsDNA substrates under the PCR system, and optimizes the enrichment conditions for mutant dsDNA, the detection results are shown in Figure 4. Enrichment conditions of mutant fragment DNA in 10nM 1.0% mut KRAS-G12D samples with PfAgo-gDNA complex under PCR system, preferably: PfAgo concentration is in the range of 20-100 nM; gDNAs concentration is in the range of 200-1000 nM; The concentration ratio of PfAgo:gDNAs is in the range of 1:10~1:20; the pretreatment time of the PfAgo-gDNA complex at 94° C is 3 minutes to 5 minutes; the number of cycles of enrichment PCR is preferably 10 to 30 cycles.

### 4.2 Results

Under the PCR system, the PfAgo-gDNA complex can differentiate and shear KRAS-G12D wild-type and mutant dsDNA substrates, thereby achieving the enrichment of mutant dsDNA.

As shown in Figure 4, after the samples after the enrichment reaction are subjected to first-generation sequencing (Sanger sequencing), and the results show that at the KRAS-G12D (gGt/gAt) mutation point position, after the 1.0% mut KRAS-G12D sample is processed, the mutation point A base has an obvious raised peak, that is, the low abundance mutation 1.0% mut KRAS-G12D DNA is significantly enriched. The enrichment factor F1b/F1a is about 78.

### Example 5.

### Enrichment and detection of low-abundance tumor gene KRAS-G12D mutant genes

### 5.1 Method

A low-abundance tumor gene KRAS-G12D mutation gene (0.1% mut, 0.01% mut) detection method. According to the experimental steps of the low-abundance mutant DNA detection system as described in the present invention, firstly, according to the sequence characteristics of the KRAS-G12D gene fragment, designing and screening specific amplification primers, gDNAs and detection probes. See the sequence in Table 2 for details.

gDNAs, primers and probes were all synthesized by Sangon Biotech (Shanghai) Co., Ltd.. KRAS gene gDNAs has a phosphorylation modification at the 5' end; the nucleotide sequence of the G12D mutant probe has a FAM fluorescent label at the 5' end, and the 3' end modifies the Quencher BHQ1; the nucleotide sequence of the KRAS wild-type gene probe is provided with a VIC fluorescent label at the 5'end, and the Quencher BHQ1 is modified at the 3' end.

This method uses the standard substance of horizon reference standards from HORIZON DISCOVERY to verify and analyze, the KRAS-G12D Expected Allelic Frequency (AF%) (mutant allele frequency (AF%)) in the standard substance is 5% mut, 1% mut, 0.1% mut, 0.01% mut and 100% wt, respectively. The 0.1% mut and 0.01% mut standard substances were used to verify the sensitivity and specificity of the low-abundance mutant DNA enrichment and detection method as described in the present invention.

The specific detection steps of the low-abundance KRAS gene mutation detection in this embodiment are as follows:

### 5.1.1. PCR pre-amplification reaction

The KRAS gene pre-amplification reaction system of this embodiment includes:
Taking the 50 µL PCR pre-amplification system as an example, pre-amplifying the 0.1% mut and 0.01% mut samples in the standard substance, respectively. The pre-amplification system is prepared as follows:
2X PCR Precision^{™} MasterMix: 25.0 µL
Forward primer (2-10 µM): 1.25 µL (SEQ ID No. 3)
Reverse primer (2-10 µM): 1.25 µL (SEQ ID No. 4)
Standard sample (0.1% mut, 0.01% mut): 2-4 µL
dd H₂O: XX µL

The volume of the reaction system can be 25.0 µL, and during the preparation, the components in the 50.0 µL reaction system can be halved.

The PCR conditions of the KRAS gene pre-amplification reaction in this embodiment are as follows:
PCR program: 94°C for 3 minutes; 10-30 cycles (94°C for 10s, 55°C for 30s, 72°C for 20s), 72°C for 1 minute.

After pre-amplification, the final product can be preliminarily quantified by TaqMan-qPCR to determine whether it meets the required target concentration in the next enrichment system.

### 5.1. 2. The PfAgo-gDNA complex enriches the low-abundance KRAS-G12D mutant genes in the pre-amplified product.

In this embodiment, the optimized enrichment conditions for KRAS-G12D mutant genes using PfAgo-gDNA complex are preferably: PfAgo concentration is 20-100 nM, gDNAs concentration is 200-2000 nM, and PfAgo:gDNAs concentration ratio is 1:5 ~ 1:20, the pretreatment time of the PfAgo-gDNA complex at 94° C is 1 minute to 5 minutes; the number of cycles of enrichment PCR is preferably 10 to 30 cycles.

The components and working conditions involved in this example mainly include: 2X PCR Precision^{™} MasterMix, forward and reverse primers, forward and reverse gDNAs, PfAgo, MnCl₂, Standard Target (0.1% mut, 0.01% mut), etc., as shown in Table 8.

For the forward and reverse primers, the preferred primers corresponding to each target gene in Table 7 were used.

For the forward and reverse gDNAs, the preferred gDNAs pairs corresponding to each target gene in Table 6 were used.

The concentration of the PfAgo mother solution stored after the pre-purification is 5µM. In actual use, it needs to be diluted in advance with the prepared Reaction Buffer under ice bath conditions: first diluting the 5µMPfAgo mother solution to 1µM, and then using Reaction Buffer to dilute it to 0.3µM. Reaction Buffer component: 15 mM Tris-Cl, 250 mM NaCl, pH 8.0.

PfAgo's PCR reaction procedures for enrichment of standards 0.1% mut, 0.01% mut KRAS-G12D mutant gene pre-amplification products include:

| step | Number of cycles | Temperature(°C) | Reaction time (Minutes: seconds) |
|---|---|---|---|
| 1 | 1 | 94 | 03:00 |
| 2 | 10-30 | 94 | 00:30 |
| | | 55 | 00:20 |
| | | 72 | 00:20 |
| 3 | 1 | 72 | 01:00 |

### 5.1.3. Detection of wild-type and mutant DNA products after enrichment of KRAS-G12D mutant genes.

The detection of the enriched product involved in the present invention can adopt various methods, such as Sanger sequencing qualitative analysis, second-generation sequencing quantitative analysis, TaqMan fluorescent quantitative PCR method quantitative analysis, fluorescence method real-time detection, high-resolution melting curve method quantitative analysis, etc.. In this embodiment, Sanger sequencing qualitative analysis and quantitative analysis by TaqMan fluorescence quantitative PCR were used to analyze the enriched products.

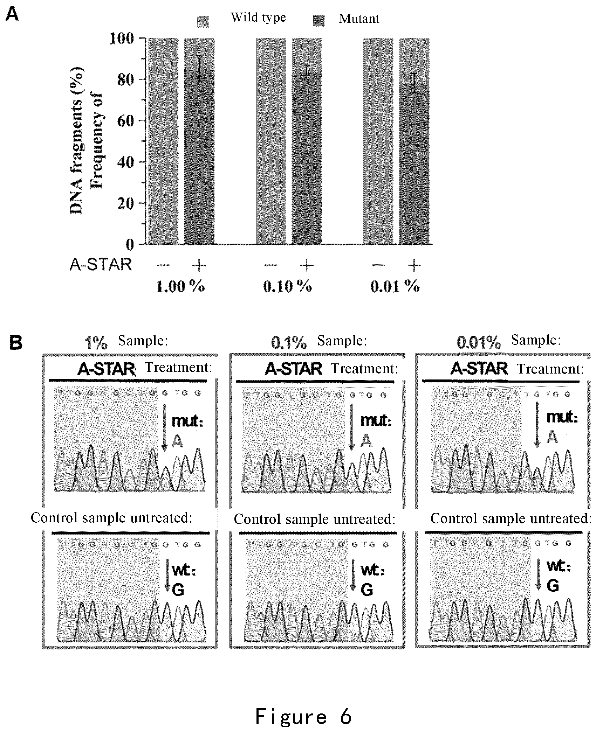

The results of first-generation sequencing (Sanger sequencing) after the enrichment reaction of the samples in this example are shown in Figure 6B. The results show that at the mutation point position of KRAS-G12D (gGt/gAt), after processing the 0.1% mut and 0.01% mut KRAS-G12D samples, the mutation point A bases have obvious raised peaks, that is, the low abundance mutations 0.1% mut and 0.01% mut KRAS-G12D DNA have been significantly enriched.

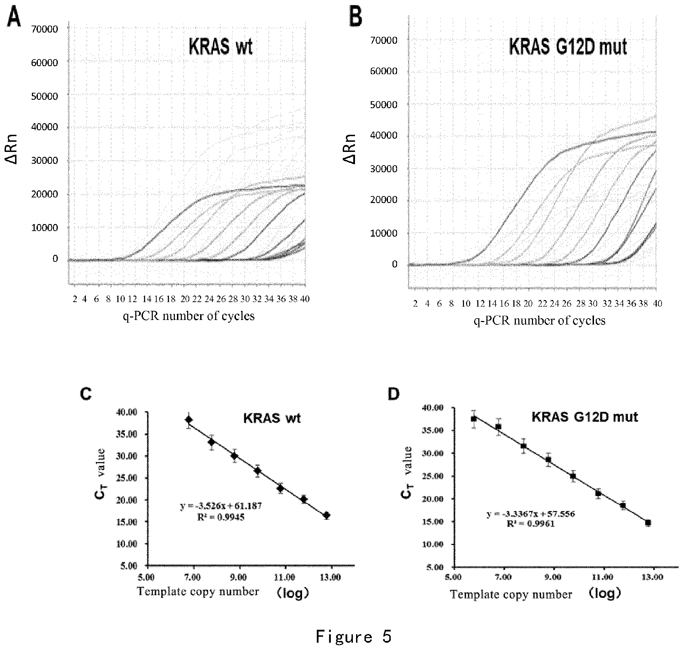

Before using the TaqMan fluorescent quantitative PCR method for quantitative analysis, the present inventor has first designed and measured the standard curve of the double TaqMan probe method for detecting the KRAS-G12D low-abundance mutant DNA (0.01%) substrate. The standard curve is shown in Figure 5.

The KRAS-G12D gene standard curve determination method of this embodiment includes:
Before the determination of the standard curve, the components are prepared in the following order:
① Template (wild type: mutant type is equal to 1:1): 158bp wt/mut KRAS G12D 10.0 pM linear gradient dilution to (10.0pM, 1.0pM, 100fM, 10fM, 1.0fM, 100aM, lOaM, 1.0aM, ddH2O)
②Dual probe: SEQ ID No.109 (10 µ M); SEQ ID No.110 (10 µ M)
③Primer pair: SEQ ID No.107 (10 µ M); SEQ ID No.108 (10 µ M)

The conditions of the TaqMan-qPCR detection system are as follows: Taking the 20 µL system as an example,
Vazayme mix (2X) 10.0 µL
Forward and reverse primers (2-10 µM) 0.5 µL each
Wild type probe (2-10 µM) 0.4 µL
Mutant probe 2-10 µM) 0.4 µL
Template (Linear gradient dilution of the sample) 3.0 µL
dd H₂O 5.2 µL

The TaqMan-qPCR program is as follows:

| step | Number of cycles | Temperature(°C) | Reaction time (Minutes: seconds) |
|---|---|---|---|
| 1 | 1 | 95 | 8:00 |
| 2 | 40 | 95 | 00:20 |
| | | 60.5 | 00:40 |

The standard curve is shown in Figure 5. Analysis of standard curve measurement results is:
① Double probe wild: mutant mother liquor is prepared at a ratio of 1:1, and the wild signal (Figure 5A) obtained from the standard sample in 10.0pM gradient dilution has good repeatability, and 3 replicates are set at each concentration. The rightmost curve in Figure 5A is the signal of the wild probe of ddH₂O. The signal of the wild probe combined with ddH₂O can be obtained. The signal threshold of the wild-type probe should be 9000. At this time, CT_{ddH2O} is about 37-39, the minimum concentration of aM-level CT of the sample is around 38, as shown in the standard curve part of Figure 5C.
②Two-probe wild: mutant mother solution is prepared in a ratio of 1:1, and the mutation signal (Figure 5B) detected by the 10.0pM gradient dilution standard sample has good reproducibility with 3 replicates set at each concentration. The rightmost curve in Figure 5B is the signal of the mutant probe of ddH₂O. The signal of the wild probe combined with ddH₂O can be obtained. The signal threshold of the wild-type probe should be 9000. At this time, CT_{ddH2O} is about 38-40, and the minimum concentration of aM-level CT of the sample is around 37, as shown in the standard curve part of Figure 5D.

After the determination of the standard curve is completed, the KRAS-G12D enriched sample of this embodiment is subjected to quantitative analysis by TaqMan fluorescent quantitative PCR method.

The components of the enriched sample are prepared in the following order before the determination:
The conditions of the TaqMan-qPCR detection system are as follows: Taking the 20 µL system as an example,
Vazayme mix (2X) 10.0 µL
Forward and reverse primers (2-10 µM) 0.5 µL each
Wild-type probe (2-10 µM) 0.4 µL
Mutant probe (2-10 µM) 0.4 µL
Template (Enriched samples were diluted 1000 times) 3.0 µL
dd H₂O 5.2 µL

The TaqMan-qPCR program is as follows:

| step | Number of cycles | Temperature(°C) | Reaction time (Minutes: seconds) |
|---|---|---|---|
| 1 | 1 | 95 | 8:00 |
| 2 | 40 | 95 | 00:15 |
| | | 61.5 | 00:40 |

### 5.2 Results

As shown in Figure 6A. In the KRAS-G12D (gGt/gAt) mutation point position, After processing 0.1% mut KRAS-G12D samples, the proportion of mutants is increased to 83% (enrichment factor F1b/F1a is 830), and after processing 0.01% mut KRAS-G12D samples, the proportion of mutants is increased to 78% (enrichment factor F1b/F1a is 7800). That is, the low abundance mutations 0.1% mut and 0.01% mut KRAS-G12D DNA are extremely enriched.

### Example 6.

### Enrichment and detection of deletion mutant genes of low-abundance tumor gene EGFR deIE746-A750 fragment

In this example, the low-abundance tumor gene EGFR delE746-A750 fragment deletion mutant gene (0.1% mut, 0.01% mut) was enriched and detected.

### 6.1 Method

According to the experimental steps of the low-abundance fragment deletion mutant DNA detection system as described in the present invention (same as in Example 5), first, according to the sequence characteristics of the EGFR deIE746-A750 gene fragment, designing and screening specific amplification primers, gDNAs and detection probes. See the sequence in Table 4 for details.

gDNAs, primers and probes were all synthesized by Sangon Biotech (Shanghai) Co., Ltd.. EGFR gene gDNAs is equipped with phosphorylation modification at the 5' end; the nucleotide sequence of delE746-A750 mutant probe is equipped with VIC fluorescent label at the 5' end, and the Quencher BHQ1 is modified at the 3' end; the 5' end of the nucleotide sequence of the wild-type EGFR gene probe is equipped with a FAM fluorescent label, and the 3' end is modified with the Quencher BHQ2.

This method uses the standard substance of horizon reference standards from HORIZON DISCOVERY to verify and analyze. The EGFR delE746-A750 Expected Allelic Frequency (AF%) mutation allele frequency (AF%) of the standard substance is 5% mut, 1% mut, 0.1% mut, 0.01% mut and 100% wt, respectively. The 0.1% mut and 0.01% mut standards were used to verify the sensitivity and specificity of the low-abundance mutant DNA enrichment and detection method as described in the present invention.

The specific detection steps of the low-abundance EGFR delE746-A750 fragment deletion mutant gene detection in this embodiment are as follows:

### 6.1.1, PCR pre-amplification reaction

The EGFR delE746-A750 gene pre-amplification reaction system of this embodiment includes:
Taking the 50 µL PCR pre-amplification system as an example, pre-amplifying the 0.1% MUT and 0.01% MUT samples in the standards. The pre-amplification system is prepared as follows:
2X PCR Precision^{™} MasterMix: 25.0 µL
FW primer (2-10 µM): 1.25 µL (SEQ ID No.23)
RV primer (2-10 µM): 1.25 µL (SEQ ID No. 24)
Standard Target (0.1% mut, 0.01% mut): 2-4 µL
dd H₂O: XX µL

The volume of the reaction system can be 25.0 µL, and the components in the 50.0 µL reaction system can be halved during preparation.

The PCR conditions of the EGFR delE746-A750 gene pre-amplification reaction in this embodiment are as follows:
PCR program: 94°C for 3 minutes; 10-30 cycles (94°C for 10s, 55°C for 30s, 72°C for 20s), 72°C for 1 minute.

After pre-amplification, the final product can be preliminarily quantified by TaqMan-qPCR to determine whether it meets the required target concentration in the next enrichment system.

### 6.1.2. PfAgo-gDNA complex enriches the low-abundance EGFR delE746-A750 mutant gene in the pre-amplified product.

In this example, the optimized enrichment conditions for EGFR delE746-A750 mutant genes using PfAgo-gDNA complex are preferably: PfAgo concentration is 20-80 nM, gDNAs concentration is 800 nM, and PfAgo:gDNAs concentration ratio is 1:5~1:20, the pretreatment time of the PfAgo-gDNA complex at 94° C is 3 minutes; the number of cycles of enrichment PCR is preferably 10 to 30 cycles.

The components and working conditions involved in this embodiment mainly include: 2×PCR Taq Master Mix, forward and reverse primers, forward and reverse gDNAs, PfAgo, MnCl₂, Standard Target (0.1% mut, 0.01% mut), etc., as shown in Table 8.

The concentration of the PfAgo mother solution stored after the pre-purification is 5µM. In actual use, it needs to be diluted in advance with the prepared Reaction Buffer under ice bath conditions: first diluting the 5µMPfAgo mother solution to 1µM, and then using the Reaction Buffer to dilute to 0.3µM.

Reaction Buffer components: 15 mM Tris-Cl, 250 mM NaCl, pH 8.0.

PfAgo's PCR reaction procedures for enrichment of pre-amplified products of standards 0.1% MUT, 0.01% MUT EGFR deIE746-A750 mutant gene include:

| step | Number of cycles | Temperature(°C) | Reaction time (Minutes: seconds) |
|---|---|---|---|
| 1 | 1 | 94 | 03:00 |
| 2 | 30 | 94 | 00:30 |
| | | 55 | 00:20 |
| | | 72 | 00:20 |

### 6.1.3. Detection of wild-type and mutant DNA products after enrichment of EGFR delE746-A750 mutant gene.

In this example, Sanger sequencing is used for qualitative analysis.

### 6.2 Results

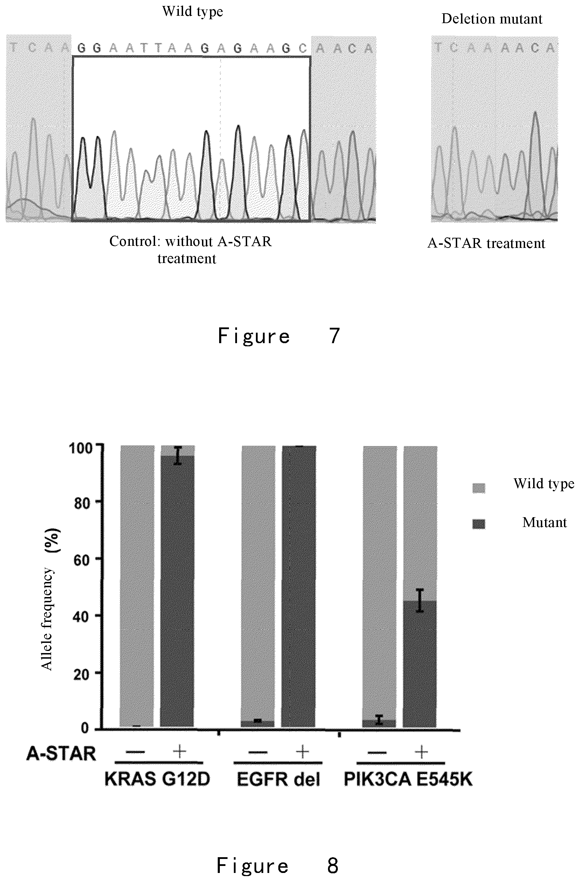

The result is shown in Figure 7. After processing the 0.1% mut and 0.01% mut samples at the EGFR delE746-A750 (fragment deletion) mutation position, showing the base sequence of the abundant missing fragments, that is, the low-abundance mutation 0.1% mut and 0.01% mut EGFR delE746-A750 DNA are significantly enriched, and the enrichment factor F1b/F1a is about 800 and 7400, respectively.

### Example 7

### Enrichment and detection of triple low-abundance mutations

In this example, multiple (triple) low abundance (0.01%mut) tumor genes KRAS-G12D, PIK3CA-E545K, and EGFR-delE746-A750 mutant genes (0.1%mut, 0.01%mut) were enriched and detected.

### 7.1 Method

According to the experimental steps of the low-abundance mutant DNA detection system described in the present invention, firstly, specific amplification primers, gDNAs and detection probes are designed and screened according to the sequence characteristics of tumor mutant gene fragments. See the sequences in Table 1, Table 2 and Table 3 for details.

gDNAs, primers and probes were all synthesized by Sangon Biotech (Shanghai) Co., Ltd.. The 5' end of gDNAs is equipped with phosphorylation modification; the 5' end of the nucleotide sequence of the probe is equipped with different fluorescent labels, and the 3' end is modified with the Quencher BHQ.

This method uses the standard substance of the horizon reference standards from HORIZON DISCOVERY to verify and analyze. The standard substance also contains the above three tumor genes. Expected Allelic Frequency (AF%) mutant allele frequencies (AF%) are 0.01% mut and 100% wt, respectively. We used a 0.01% mut standards to verify the sensitivity and specificity of the triple low-abundance mutant DNA enrichment and detection method as described in this invention.

The specific detection steps of the triple low-abundance tumor gene mutation detection in this embodiment are as follows:

### 7.1.1. PCR pre-amplification reaction

The tumor gene pre-amplification reaction system of this embodiment includes:
Taking the 50 µL PCR pre-amplification system as an example, pre-amplifying 0.01% MUT samples in the standards. The pre-amplification system is prepared as follows:
2X PCR Precision^{™} Master Mix: 25.0 µL
Forward primer (10 µM): 1.25 µL (SEQ ID No.3, SEQ ID No.13, SEQ ID No.23)
Reverse primer (10 µM): 1.25 µL (SEQ ID No. 4, SEQ ID No. 14, SEQ ID No. 24)
Standard substance (0.1% mut, 0.01% mut): 1-2 µL
dd H₂O: XX µL

The volume of the reaction system can be 25.0 µL, and the components in the 50.0 µL reaction system can be halved during preparation.

The PCR conditions of the triple low-abundance tumor gene pre-amplification reaction of this embodiment are as follows:
PCR program: 94°C for 3 minutes; 24-30 cycles (94°C for 30s, 55°C for 30s, 72°C for 20s); 72°C for 1 minute.

After pre-amplification, the final product can be preliminarily quantified by TaqMan-qPCR to determine whether it meets the required target concentration in the next enrichment system.

### 7.1.2. PfAgo-gDNA complex enrichment of triple low-abundance tumor mutant genes in pre-amplified products.

In this example, the optimized enrichment conditions for KRAS-G12D, PIK3CA-E545K and EGFR-delE746-A750 mutant genes using PfAgo-gDNA complex are preferably: the PfAgo concentration is 10-800 nM, and the gDNAs concentration is 100- 4000nM; PfAgo:gDNAs concentration ratio is 1:5~1:20, the pretreatment time of the PfAgo-gDNA complex at 94° C is 3 minutes; the number of cycles of enrichment PCR is preferably 10 to 30 cycles.

The components and working conditions involved in this example mainly include: 2X PCR Precision^{™} MasterMix, forward and reverse primers, forward and reverse gDNAs, PfAgo, MnCl₂, Standard Target (0.01% mut), etc., as shown in Table 8.

For the forward and reverse primers, the preferred primers corresponding to each target gene in Table 7 were used.

For the forward and reverse gDNAs, the preferred gDNAs pairs corresponding to each target gene in Table 6 were used.

The concentration of the PfAgo mother solution stored after the pre-purification is 5µM. In actual use, it should be diluted with the prepared Reaction Buffer in advance under ice bath conditions: first diluting the 5µM PfAgo mother solution to 1µM, and then diluting it to 0.3 µM with Reaction Buffer.

Reaction Buffer components: 15 mM Tris-Cl, 250 mM NaCl, pH 8.0.

PfAgo's PCR reaction procedures for enrichment of the pre-amplified product of standards 0.01% mut KRAS-G12D mutant gene includes:

| step | Number of cycles | Temperature(°C) | Reaction time (Minutes: seconds) |
|---|---|---|---|
| 1 | 1 | 94 | 03:00 |
| 2 | 10-30 | 94 | 00:30 |
| | | 55 | 00:20 |
| | | 72 | 00:20 |
| 3 | 1 | 72 | 01:00 |

### 7.1.3. Detection of wild-type and mutant DNA products after enrichment of KRAS-G12D, PIK3CA-E545K and EGFR-delE746-A750 mutant genes.

The detection of the enriched product involved in the present invention can adopt various methods, such as Sanger sequencing qualitative analysis, second-generation sequencing quantitative analysis, TaqMan fluorescent quantitative PCR method quantitative analysis, fluorescence method real-time detection, high-resolution melting curve method quantitative analysis, etc.. In this example, the TaqMan fluorescent quantitative PCR method was used to analyze the enriched products.

Before using the TaqMan fluorescent quantitative PCR method for quantitative analysis, we first designed and measured the standard curve of the double TaqMan probe method for detecting KRAS-G12D, PIK3CA-E545K and EGFR-delE746-A750 low-abundance mutant DNA substrates.

After the determination of the standard curve, the KRAS-G12D, PIK3CA-E545K and EGFR-delE746-A750 enriched samples of this example were subjected to TaqMan fluorescent quantitative PCR method for quantitative analysis.

The components of the enriched sample are prepared in the following order before the determination:
The conditions of the TaqMan-qPCR detection system are as follows: Taking the 20 µL system as an example,
Vazayme mix (2X) 10.0 µL
Forward and reverse primers (10 µM) 0.5 µL each
Wild type probe (10 µM) 0.4 µL
Mutant probe (10 µM) 0.4 µL
Template (Enriched samples were diluted 1000 times) 3.0 µL
dd H₂O 5.2 µL

The TaqMan-qPCR program is as follows:

| step | Number of cycles | Temperature(°C) | Reaction time (Minutes: seconds) |
|---|---|---|---|
| 1 | 1 | 95 | 8:00 |
| 2 | 40 | 95 | 00:20 |
| | | 60 | 00:40 |

### 7.2 Results

As shown in Figure 8, in the KRAS-G12D, PIK3CA-E545K and EGFR-delE746-A750 mutation positions, after processing 0.01% mut samples, the proportion of mutant KRAS-G12D, EGFR-delE746-A750 and PIK3CA-E545K is increased to 79%, 79%, and 41%, respectively. That is, the triple low-abundance mutation 0.01% mut tumor gene has also been significantly enriched, and the enrichment factor F1b/F1a is about 7900, 7900 and 4100, respectively.

**Table 1**

| Mutation site | KRAS G12D | PIK3CA E545K | EGFR-delE746-A750 | NRAS A59T |
|---|---|---|---|---|
| Primer pair | SEQ ID No. 3 | SEQ ID No. 13 | SEQ ID No.23 | SEQ ID No.33 |
| | SEQ ID No. 4 | SEQ ID No.14 | SEQ ID No.24 | SEQ ID No.34 |
| gDNA pair | SEQ ID No. 5 | SEQ ID No.15 | SEQ ID No.25 | SEQ ID No.35 |
| | SEQ ID No. 6 | SEQ ID No.16 | SEQ ID No.26 | SEQ ID No.36 |
| Wild-type and mutant gene detection probes | SEQ ID No. 9 | SEQ ID No.19 | SEQ ID No.29 | SEQ ID No.39 |
| | SEQ ID No.10 | SEQ ID No.20 | SEQ ID No.30 | SEQ ID No.40 |

**Table 2**

| KRAS G12D (gGt/gAt) | sequence (5'-3' ) | SEQ ID No. |
|---|---|---|
| KRAS G12D(wt) totalLen= 15 8 | GTGACATGTTCTAATATAGTCACATTTTCATTAT TTTTATTATAAGGCCTGCTGAAAATGACTGAAT ATAAACTTGTGGTAGTTGGAGCTGGTGGCGTAG GCAAGAGTGCCTTGACGATACAGCTAATTCAG | SEQ ID No. 1 |
| | AATCATTTTGTGGACGAATATGATCC | |
| KRAS G12D(mut) totalLen= 15 8 | GTGACATGTTCTAATATAGTCACATTTTCATTAT TTTTATTATAAGGCCTGCTGAAAATGACTGAAT ATAAACTTGTGGTAGTTGGAGCTGATGGCGTAG GCAAGAGTGCCTTGACGATACAGCTAATTCAG AATCATTTTGTGGACGAATATGATCC | SEQ ID No.2 |
| KRAS G12D-158F | 5'-GTGACATGTTCTAATATAGTC-3' | SEQ ID No.3 |
| KRAS G12D-158R | 5'-GGATCATATTCGTCCACAAA-3' | SEQ ID No. 4 |
| KRAS G12D-GF1 | 5'-P-TTTGGAGCTAGTGGCG-P-3'(KRAS-G12D FW-10A) | SEQ ID No. 5 |
| KRAS G12D-GR1 | 5'-P-TCTACGCCACAAGCTC-P-3'(KRAS-G12D RV-11A) | SEQ ID No. 6 |
| KRAS G12D-7PF | 5'-AGGCCTGCTGAAAATGACTG-3' | SEQ ID No. 7 |
| KRAS G12D-7PR | 5'-GCTGTATCGTCAAGGCACTCT-3' | SEQ ID No. 8 |
| KRAS G12D(wt)-7P | TTGGAGCTGGTGGCGTA (VIC-BHQ1) | SEQ ID No.9 |
| KRAS G12D(mut)-7 P | TTGGAGCTGATGGCGTA (FAM-BHQ1) | SEQ ID No. 10 |
| KRAS G12D qPCR Product(80 bp) | AGGCCTGCTGAAAATGACTGAATATAAACTTGT GGTAGTTGGAGCTGGTGGCGTAGGCAAGAGTG CCTTGACGATACAGC | |

F: forward primer; R: reverse primer; P: TaqMan-MGB probe; GF: Guide Forward DNA; GR: Guide Reverse DNA

**Table 3**

| PIK3CA E545K (Gag/ Aag) | sequence (5'-3' ) | SEQ ID No. |
|---|---|---|
| PIK3CA E545K(wt) totalLen=139 | GAGACAATGAATTAAGGGAAAATGACAAA GAACAGCTCAAAGCAATTTCTACACGAGAT CCTCTCTCTGAAATCACTGAGCAGGAGAAA GATTTTCTATGGAGTCACAGGTAAGTGCTA AAATGGAGATTCTCTGTTTC | SEQ ID No.11 |
| PIK3CA E545K(mut) totalLen=139 | GAGACAATGAATTAAGGGAAAATGACAAA GAACAGCTCAAAGCAATTTCTACACGAGAT CCTCTCTCTGAAATCACTAAGCAGGAGAAA GATTTTCTATGGAGTCACAGGTAAGTGCTA AAATGGAGATTCTCTGTTTC | SEQ ID No.12 |
| PIK3CA E545K-139F | 5'-GAGACAATGAATTAAGGGAA-3' | SEQ ID No.13 |
| PIK3CA E545K-139R | 5'-GAAACAGAGAATCTCCATT-3' | SEQ ID No.14 |
| PIK3CA E545K-GF1 | 5' - P- TGAAA TCACCGAGCAG- P-3 '(PIK3 CA- E54 5K FW-10C) | SEQ ID No.15 |
| PIK3CA E545K-GR1 | 5'-P-TTCTCCTGCGCAGTGA-P-3'(PIK3CA-E545 K RV-10G) | SEQ ID No.16 |
| PIK3CA E545K-4PF | 5'-GAACAGCTCAAAGCAATTTCTACAC-3' | SEQ ID No.17 |
| PIK3CA E545K-4PR | 5'-AGCACTTACCTGTGACTCCATAG-3' | SEQ ID No.18 |
| PIK3CA E545K(wt)-4P | CTGAAATCACTGAGCAGGA(FAM-BHQ1) | SEQ ID No.19 |
| PIK3CA E545K(mut)-4P | TCTGAAATCACTAAGCAGGA (VIC-BHQ 1) | SEQ ID No.20 |
| PIK3CA E545K qPCR | GAACAGCTCAAAGCAATTTCTACACGAGAT CCTCTCTCTGAAATCACTGAGCAGGAGAAA | |
| Product(89 bp) | GATTTTCTATGGAGTCACAGGTAAGTGCT | |

F: forward primer; R: reverse primer; P: TaqMan-MGB probe; GF: Guide Forward DNA; GR: Guide Reverse DNA

**Table 4**

| EGFR delE746-A750( f ragment deletion) | sequence (5'-3' ) | SEQ ID No. |
|---|---|---|
| EGFR delE746-A750 (wt) totalLen=157 | CTGTCATAGGGACTCTGGATCCCAGAA GGTGAGAAAGTTAAAATTCCCGTCGCT ATCAAGGAATTAAGAGAAGCAACATCT CCGAAAGCCAACAAGGAAATCCTCGAT GTGAGTTTCTGCTTTGCTGTGGGGGT CCATGGCTCTGAACCTCAGGC | SEQ ID No.21 |
| EGFR delE746-A750 (mut) totalLen=142 | CTGTCATAGGGACTCTGGATCCCAGAA GGTGAGAAAGTTAAAATTCCCGTCGCT ATCAAAACATCTCCGAAAGCCAACAAG GAAATCCTCGATGTGAGTTTCTGCTTTG CTGTGTGGGGGTCCATGGCTCTGAACC TCAGGC | SEQ ID No.22 |
| EGFR delE746-157F | 5'-CTGTCATAGGGACTCTGGAT-3' | SEQ ID No.23 |
| EGFR delE746-157R | 5'-GCCTGAGGTTCAGAGCCAT-3' | SEQ ID No.24 |
| EGFR delE746-A750-G F1 | 5'-P-TAAGGAATTAAGAGAA-P-3'(EGFR-Del-FW) | SEQ ID No.25 |
| EGFR delE746-A750-G R1 | 5'-P-TTGCTTCTCTTAATT-P-3'(EGFR-Del-RV) | SEQ ID No.26 |
| EGFR delE746-A750-4 PF | 5'-CCAGAAGGTGAGAAAGTTA-3' | SEQ ID No.27 |
| EGFR delE746-A750-4 PR | 5'-TCGAGGATTTCCTTGTTG-3' | SEQ ID No.28 |
| EGFR delE746-A750(w t)-4P | CTTCTCTTAATTCCTTGATAGCGACGG (FAM-BHQ2) | SEQ ID No.29 |
| EGFR delE746-A750(m ut)-4P | CGCTATCAAAACATCTCCGAAAGCC (VIC-BHQ1) | SEQ ID No.30 |
| EGFR delE746-A750 qPCR Product (86/71 bp) | CCAGAAGGTGAGAAAGTTAAAATTCCC GTCGCTATCAAGGAATTAAGAGAAGCA ACATCTCCGAAAGCCAACAAGGAAATC CTCGA/CCAGAAGGTGAGAAAGTTAAA ATTCCCGTCGCTATCAAAACATCTCCGA AAGCCAACAAGGAAATCCTCGA | |

F: forward primer; R: reverse primer; P: TaqMan-MGB probe; GF: Guide Forward DNA; GR: Guide Reverse DNA

**Table 5**

| NRAS A59T(Caa/Aaa) | sequence (5'-3' ) | SEQ ID No. |
|---|---|---|
| NRAS A59T (wt) totalLen=154 | CCAGGATTCTTACAGAAAACAAGTGGTTA TAGATGGTGAAACCTGTTTGTTGGACATA CTGGATACAGCTGGACAAGAAGAGTACA GTGCCATGAGAGACCAATACATGAGGAC AGGCGAAGGCTTCCTCTGTGTATTTGCCA TCAATAATAGC | SEQ ID No.31 |
| NRAS A59T | CCAGGATTCTTACAGAAAACAAGTGGTTA | SEQ ID No.32 |
| (mut) totalLen=154 | TAGATGGTGAAACCTGTTTGTTGGACATA CTGGATACAGCTGGAAAAGAAGAGTACA GTGCCATGAGAGACCAATACATGAGGAC AGGCGAAGGCTTCCTCTGTGTATTTGCCA TCAATAATAGC | |
| NRAS A59T-154F | 5'-CCAGGATTCTTACAGAAAACAAGT-3' | SEQ ID No.33 |
| NRAS A59T-154R | 5'-GCTATTATTGATGGCAAATACACAG-3' | SEQ ID No.34 |
| NRAS A59T-GF1 | 5'-P-TTGGATACAGTTGGAC-P-3'(NRAS-A59 T FW-11T) | SEQ ID No.35 |
| NRAS A59T-GR1 | 5'-P-TCTTGTCCATCTGTAT-P-3'(NRAS-A59 T RV-10T) | SEQ ID No.36 |
| NRAS A59T-PF | 5'-GATGGTGAAACCTGTTTGTTGGA-3' | SEQ ID No.37 |
| NRAS A59T-PR | 5'-TCGCCTGTCCTCATGTATTGG-3' | SEQ ID No.38 |
| NRAS A59T(wt)-P | CTGGATACAGCTGGACAA (VIC-BHQ1) | SEQ ID No.39 |
| NRAS A59T(mut)-P | TACTGGATACAACTGGACAA (FAM-BHQ2) | SEQ ID No.40 |
| NRAS A59T qPCR Product (90bp) | AGATGGTGAAACCTGTTTGTTGGACATAC TGGATACAGCTGGACAAGAAGAGTACAG TGCCATGAGAGACCAATACATGAGGACA GGCGA | |

F: forward primer; R: reverse primer; P: TaqMan-MGB probe; GF: Guide Forward DNA; GR: Guide Reverse DNA

**Table 6**

| Target | gDNA sequence (5'-3') | SEQ ID No. |
|---|---|---|
| KRAS G12D-GF1 | 5'-P-TTTGGAGCTAGTGGCG-P-3'(KRAS-FW-10A) | SEQ ID No.5 |
| KRAS G12D-GR1 | 5'-P-TCTACGCCACAAGCTC-P-3'(KRAS-RV-11A) | SEQ ID No.6 |
| PIK3CA E545K-GF1 | 5'-P-TGAAATCACCGAGCAG-P-3'(PIK3CA-FW-10C) | SEQ ID No.15 |
| PIK3CA E545K-GR1 | 5'-P-TTCTCCTGCGCAGTGA-P-3'(PIK3CA-RV-10G) | SEQ ID No.16 |
| EGFR delE746-A750-GF1 | 5'-P-TAAGGAATTAAGAGAA-P-3'(EGFR-Del-FW) | SEQ ID No.25 |
| EGFR delE746-A750-GR1 | 5'-P-TTGCTTCTCTTAATT-P-3'(EGFR-Del-RV) | SEQ ID No.26 |
| NRAS A59T-GF1 | 5'-P-TTGGATACAGTTGGAC-P-3'(NRAS-A59T FW-11T) | SEQ ID No.35 |
| NRAS A59T-GR1 | 5'-P-TCTTGTCCATCTGT AT -P-3'(NRAS-A59T RV-10T) | SEQ ID No.36 |

**Table 7**

| Target | Primer sequence (5'-3' ) | SEQ ID No. |
|---|---|---|
| KRAS G12D-158F | 5'-GTGACATGTTCTAATATAGTC-3' | SEQ ID No.3 |
| KRAS G12D-158R | 5'-GGATCATATTCGTCCACAAA-3' | SEQ ID No.4 |
| PIK3CA E545K-139F | 5'-GAGACAATGAATTAAGGGAA-3' | SEQ ID No.13 |
| PIK3CA E545K-139R | 5'-GAAACAGAGAATCTCCATT-3' | SEQ ID No.14 |
| EGFR delE746-157F | 5'-CTGTCATAGGGACTCTGGAT-3' | SEQ ID No.23 |
| EGFR delE746-157R | 5'-GCCTGAGGTTCAGAGCCAT-3' | SEQ ID No.24 |
| NRAS A59T-154F | 5'-CCAGGATTCTTACAGAAAACAAGT-3' | SEQ ID No.33 |
| NRAS A59T-154R | 5'-GCTATTATTGATGGCAAATACACAG-3' | SEQ ID No.34 |

**Table 8. PfAgo enrichment reaction 25 µL system components and preparation sequence**

| **loading order** | **Component** | **volume** |
|---|---|---|
| 1 | 2× PCR Taq Master Mix | 12.5 µL |
| 2 | dd H₂O | 3.75 µL |
| 3 | FW/RV primer (10 µM) | each 0.5 µL |
| 4 | MnCl₂ (10 mM) | 1.25 µL |
| 5 | Template | 2 µL |
| 6 | Ago (XX nM) | X µL |
| 7 | gDNA mix | X µL |

| | | |
|---|---|---|
| Note: Please add the above samples in order when preparing the system. | | |

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

The specific embodiments described above further describe the technical problems, technical solutions and beneficial effects solved by the present invention in further detail. It should be understood that the above descriptions are only specific embodiments of the present invention and are not intended to limit In the present invention, any modification, equivalent replacement, improvement, etc. made within the spirit and principle of the present invention shall be included in the protection scope of the present invention.

## Claims

1. A method for increasing the relative abundance of target nucleic acid, comprising the steps:
(a) providing a nucleic acid sample, the nucleic acid sample contains a first nucleic acid and a second nucleic acid, wherein the first nucleic acid is a target nucleic acid and the second nucleic acid is a non-target nucleic acid,
and, the abundance of the target nucleic acid in the nucleic acid sample is F1a;
(b) performing a polymerase chain reaction (PCR) and nucleic acid cleavage reaction in an amplification-cleavage reaction system using the nucleic acid in the nucleic acid sample as a template, thereby obtaining an amplification-cleavage reaction product;
wherein, the nucleic acid cleavage reaction is used to specifically cleave the non-target nucleic acid, but not the target nucleic acid;
in addition, the amplification-cleavage reaction system contains (i) a reagent required for PCR reaction and (ii) a reagent required for nucleic acid cleavage reaction;
wherein, the abundance of the target nucleic acid in the amplification-cleavage reaction product is Fib,
wherein the ratio of F1b/ F1a is ≥ 10.

2. The method of claim 1, wherein the target nucleic acid and the non-target nucleic acid differ by only one base.

3. The method of claim 1, wherein the target nucleic acid is a nucleotide sequence containing a mutation.

4. The method of claim 1, wherein the nucleic acid cleavage tool enzyme is selected from but not limited to the following Argonaute proteins and the mutants thereof from Thermophiles (≥60° C): *Pf*Ago (*Pyrococcus furiosus* Ago), *Mƒ*Ago (*Methanocaldococcus fervens* Ago), *Tc*Ago (*Thermogladius calderae* Ago), *Tf*Ago (*Thermus ƒiliƒormis* Ago), *Aa*Ago (*Aquifex aeolicus* Ago), etc..

5. The method of claim 1, wherein the gDNA and the nucleic acid sequence in the target region of the target nucleic acid (i.e., a first nucleic acid) form a first complementary binding region; and the gDNA also forms a second complementary binding region with the nucleic acid sequence of the target region of the non-target nucleic acid (i.e., a second nucleic acid).

6. The method of claim 1, wherein the ratio (molar ratio) of the nucleic acid cleavage tool enzyme and gDNAs is 1:2 to 1:20.

7. The method of claim 1, wherein the method further includes:
(c) detecting the amplification-cleavage reaction product, thereby determining the presence and/or quantity of the target nucleic acid.

8. The method of claim 1, wherein the first nucleic acid includes n different nucleic acid sequences, wherein n is a positive integer ≥ 1.

9. The method of claim 1, wherein in step (b), C cycles of "high temperature denaturation-extension" are performed, wherein C is ≥ 5.

10. An amplification-cleavage reaction system, which is used to simultaneously perform a polymerase chain reaction (PCR) and nucleic acid cleavage reaction on a nucleic acid sample, thereby obtaining an amplification-cleavage reaction product;
wherein, the nucleic acid sample contains a first nucleic acid and a second nucleic acid, wherein the first nucleic acid is a target nucleic acid, and the second nucleic acid is a non-target nucleic acid;
the nucleic acid cleavage reaction is used to specifically cut the non-target nucleic acid, but not the target nucleic acid;
the amplification-cleavage reaction system contains (i) a reagent required for PCR reaction and (ii) a reagent required for nucleic acid cleavage reaction.
